# EUROPÄISCHE PATENTSCHRIFT

(11) **EP 3 481 280 B1**
(45) Veröffentlichungstag und Bekanntmachung des Hinweises auf die Patenterteilung: **02.09.2020**
(21) Anmeldenummer: 17737766.0
(22) Anmeldetag: 04.07.2017
(51) Int. Cl.: A61B 5/00, A61B 5/103, A61Q 5/00

(54) **VERFAHREN ZUM ERMITTELN EINER NUTZERSPEZIFISCHEN HAARBEHANDLUNG**
METHOD FOR ESTABLISHING A USER-SPECIFIC HAIR CARE TREATMENT
PROCÉDÉ POUR ÉTABLIR UN TRAITEMENT CAPILLAIRE SPÉCIFIQUE POUR UN UTILISATEUR

(30) Priorität: 05.07.2016 DE 102016212202; 01.12.2016 DE 102016223916
(43) Veröffentlichungstag der Anmeldung: 15.05.2019
(73) Patentinhaber: Henkel AG & Co. KGaA, 40589 Düsseldorf (DE)
(72) Erfinder: KNÜBEL, Georg, 40219 Düsseldorf (DE); GEBERT-SCHWARZWÄLDER, Antje, 41469 Neuss (DE); FÖRSTER,Thomas, 40591 Düsseldorf (DE)
(86) Internationale Anmeldenummer: PCT/EP2017/066579
(87) Internationale Veröffentlichungsnummer: WO 2018/007357

(56) Entgegenhaltungen:
- WO-A1-2015/166403
- US-A1- 2002 010 556
- US-A1- 2006 281 994
- US-A1- 2014 118 521
- US-A1- 2016 175 620

## Beschreibung

Die Erfindung betrifft ein Verfahren zum Ermitteln einer nutzerspezifischen Haarbehandlung.

Das menschliche Haar besteht aus Proteinen, Lipiden, Wasser, Spurenelementen und Pigmenten. Das Faserprotein Keratin ist der Hauptbestandteil der Haare. Die Lipide, die im menschlichen Haar vorhanden sind, sind entweder frei oder kovalent gebunden. Melanine sind rötliche, braune oder schwarze Pigmente, die die Färbung der Haare bewirken.

Die quantitative Bestimmung der Aminosäuren, kovalent gebundener Lipide und der Melanine erfordert die Solubilisierung oder Totalhydrolyse des Keratins. Dies kann beispielsweise mittels saurer oder alkalischer Totalhydrolyse oder mittels enzymatischen Abbaus erfolgen. Mittels chromatografischer Verfahren, wie beispielsweise Hochleistungsflüssigkeitschromatografie (HPLC), Gaschromatografie (GC), Dünnschichtchromatografie (DC) oder lonenaustauschchromatografie (IC) kann der Gehalt der einzelnen Aminosäuren, Lipide oder Melanine bestimmt werden.

Die Bestimmung des Wassergehaltes in Haaren kann beispielsweise mittels Karl-Fischer-Titration erfolgen.

Umwelteinflüsse (insbesondere UV-Lichtbestrahlung), kosmetische Behandlungen (beispielsweise Blondierungen, Dauerwellen oder Hitzebehandlungen) oder bestimmte Diäten (insbesondere Protein-arme Diäten) führen dazu, dass sich die Zusammensetzung in Bezug auf einen oder mehrere Inhaltsstoff(e) der Haare ungünstig verändert.

Dies kann dazu führen, dass den Haaren Glanz und Halt fehlt oder dass sie sich trocken und spröde anfühlen und dadurch schwer zu handhaben sind.

Es gibt viele verschiede Haarpflegeprodukte am Markt, welche unterschiedliche Haareigenschaften oder -parameter, wie beispielsweise den Glanz, verbessern sollen. In vielen Fällen weiß der Anwender solcher Produkte aber nicht, in welchem Zustand seine Haare sind. Er weiß beispielsweise oftmals nicht, wie stark und auf welche Weise seine Haare geschädigt sind. Dies kann dazu führen, dass der Anwender zu in seinem speziellen Fall weniger geeigneten Produkten greift und nach ihrer Anwendung mit deren Wirksamkeit unzufrieden ist.

Deshalb kann eine Ermittlung der Zusammensetzung des Haars in Bezug auf die enthaltenen Aminosäuren, Lipide und Melanine sowie des Wassergehaltes von großer Bedeutung sein.

Wie oben beschrieben, steht einem Forscher im akademischen und industriellen Bereich eine Vielzahl von physikalischen und chemisch-analytischen Verfahren zur Verfügung, um eine Ermittlung der Zusammensetzung von Haaren durchzuführen.

Allerdings sind all diese Verfahren kompliziert und apparativ sowie zeitlich aufwändig, so dass sie einem Endverbraucher nicht zur Verfügung stehen.

Die Dokumente US2006/281994 A1 und US 2002/010556 A1 offenbaren Verfahren zur Bestimmung der Haarschädigung. Das Dokument WO 2015/166403 A1 beschreibt ein Verfahren zur Bestimmung der Zusammensetzung eines Haarfärbungsmittels. Das Dokument US 2016/175620 A1 beschreibt ein Verfahren zur Haarbehandlung, wobei die Haarbehandlung auf einem bestimmten Zustand des Haares basiert.

Immer mehr Anwender von Produkten wünschen sich ein auf ihre individuellen Bedürfnisse abgestimmtes Produkt. Dies gilt insbesondere auch für Schönheitsprodukte wie Haut- und/oder Haarbehandlungsmittel.

Es war deshalb eine Aufgabe der vorliegenden Erfindung ein Verfahren zur Ermittlung individueller Haarbehandlungsanweisungen bereitzustellen, welches einem Endverbraucher auf einfache Weise erlaubt, den Gehalt mindestens eines Inhaltsstoffes seiner Haare zu bestimmen und eine darauf abgestimmte Haarbehandlungsanweisung zu erhalten.

Diese Aufgabe wird gelöst durch ein Verfahren zur Ermittlung einer individualisierten Haarbehandlung mit den Schritten:
a) Ermitteln eines Gehalts an Haarinhaltsstoff mehrerer Proben von Haaren mit einem unterschiedlichen Haarzustand mittels eines chromatografischen oder colorimetrischen Verfahrens;
b) Aufnahme von Nahinfrarot- und/oder Infrarot- und/oder VIS- und/oder Raman-Spektren der Proben von Haaren mit einem unterschiedlichen Haarzustand;
c) Erstellen eines Kalibriermodells, das eine Korrelation zwischen Nahinfrarot- und/oder Infrarot- und/oder VIS- und/oder Raman-Spektren und dem Gehalt an Haarinhaltsstoff herstellt;
d) Aufnahme von einem Nahinfrarot- und/oder einem Infrarot- und/oder einem VIS- und/oder einem Raman-Spektrum von Haaren eines Individuums;
e) Bestimmung eines Haarzustands der Haare dieses Individuums anhand des Kalibriermodells; und
f) Ausgabe einer individuellen Behandlungsanweisung zu den Haaren des Individuums in Abhängigkeit vom ermittelten Haarzustand,
wobei der Haarinhaltsstoff ausgewählt ist aus der Gruppe bestehend aus Aminosäuren, Lipiden, Melaninen, Wasser und Mischungen davon.

Der Begriff Haare umfasst im Rahmen dieser Anmeldung keratinhaltige Fasern wie Pelze, Wolle und Federn, insbesondere aber menschliche Haare.

Die Nahinfrarot- und/oder Infrarot- und/oder VIS- und/oder Raman-Spektroskopie erlaubt die direkte, zerstörungsfreie Bestimmung des Gehalts an einem Haarinhaltsstoff ohne eine aufwendige

Probenvorbereitung und ohne das Haar durch die Analyse in seiner Struktur zu verändern oder zu zerstören. Dies hat den Vorteil, dass das Ergebnis sehr schnell erhalten wird und die Haare gleich nach der Bestimmung des Gehalts an Haarinhaltsstoff einer Behandlung unterzogen werden können. Ein weiterer Vorteil ist, dass die Nahinfrarot- und/oder Infrarot- und/oder VIS- und/oder Raman-Spektroskopie an Haaren, welche sich am Kopf des Individuums befinden, ausgeführt werden kann.

Menschliche Haare enthalten neben den 20 kanonischen Aminosäuren Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin und Trypthophan ferner die Aminosäuren Cystin, Ornithin und Citrullin. Entsprechend ist es bevorzugt, dass der Haarinhaltsstoff, dessen Gehalt bestimmt wird, eine Aminosäure ausgewählt aus der Gruppe bestehend aus Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin, Trypthophan, Cystin, Ornithin, Citrullin und Mischungen davon ist.

Es ist insbesondere bevorzugt, dass der Haarinhaltsstoff, dessen Gehalt bestimmt wird, eine Aminosäure ausgewählt aus der Gruppe bestehend aus Cystein, Methionin, Glutamin, Arginin, Tyrosin, Cystin und Mischungen ist. Der Gehalt dieser Aminosäuren, insbesondere an der L-Form dieser Aminosäuren, hat einen großen Einfluss auf den Zustand von Haaren.

Lipide, welche in menschlichen Haaren enthalten sind, umfassen Kohlenwasserstoffe, Squalen, Wachsester, Triglyceride, Fettsäuren, Cholesterol, Cholesterolsulfat, Ceramide und 18-Methyleicosansäure. Entsprechend ist es bevorzugt, dass der Haarinhaltsstoff, dessen Gehalt bestimmt wird, ein Lipid ausgewählt aus der Gruppe bestehend aus Kohlenwasserstoffen, Squalen, Wachsestern, Triglyceriden, Fettsäuren, Cholesterol, Cholesterolsulfat, Ceramiden, 18-Methyleicosansäure (18-MEA) und Mischungen davon ist.

Es ist insbesondere bevorzugt, dass der Haarinhaltsstoff, dessen Gehalt bestimmt wird, ein Lipid umfasst, welches 18-Methyleicosansäure ist.

Melanin tritt beim Menschen vor allem in zwei Varianten auf: eine braun-schwärzliche (Eumelanin), die sich von den Aminosäuren Tyrosin und Levodopa ableitet und eine hellere gelblich-rötliche (Phäomelanin) Variante, die schwefelhaltig ist. Entsprechend ist es bevorzugt, dass der Haarinhaltsstoff, dessen Gehalt bestimmt wird, ein Melanin ausgewählt aus der Gruppe bestehend aus Eumelanin, Phäomelanin und Mischungen davon ist.

In einer ganz besonders bevorzugten Ausführungsform der Erfindung wird der Gehalt von zwei oder mehr Haarinhaltsstoffen bestimmt. Die zwei oder mehr Haarinhaltsstoffe sind ausgewählt aus der

Gruppe bestehend aus Aminosäuren, Lipiden, Melaninen, Wasser und Mischungen daraus. Die zwei oder mehr Haarinhaltsstoffe können dabei aus einer Verbindungsklasse oder mehreren Verbindungsklassen ausgewählt sein. Dies bedeutet, dass der Gehalt von zwei oder mehr Aminosäure, von zwei oder mehr Lipiden oder von Eumelanin und Phäomelanin bestimmt werden kann. Alternativ können die zwei oder mehr Haarinhaltsstoffe aus unterschiedlichen Verbindungsklassen ausgewählt sein. So können beispielsweise die Gehalte von einer oder mehr Aminosäure(n) und einem oder mehr Lipid(en) bestimmt werden. Alternativ können die Gehalte von einer oder mehr Aminosäure(n) und einem oder mehr Melanin(en) bestimmt. In einer weiteren Alternative werden die Gehalte von einem oder mehr Lipid(en) und einem oder mehr Melanin(en) bestimmt. In noch einer weiteren Alternative werden die Gehalte von einer oder mehr Aminosäure(n), einem oder mehr Lipid(en) und einem oder mehr Melanin(en) bestimmt.

In noch einer weiteren Alternative ist ein Haarinhaltsstoff Wasser und zusätzlich zum Wassergehalt wird/werden der/die Gehalt(e) von einer oder mehreren Aminosäure(n), einem oder mehreren Lipid(en) und/oder einem oder zwei Melanin(en) bestimmt.

Das beanspruchte Verfahren verlangt zunächst die Erstellung eines Kalibriermodells. Dazu wird in einem Schritt a) zunächst der Gehalt eines Haarinhaltsstoffes mehrerer Proben in unterschiedlichen Zuständen mittels eines unabhängigen, zum Beispiel eines chromatografischen, beispielsweise HPLC, oder eines colorimetrischen, Verfahrens bestimmt. Der Gehalt an Wasser in Haarproben kann beispielsweise mittels des Mikrowellen-Messverfahrens bestimmt werden.

In einem Schritt b) werden dann Nahinfrarot- und/oder Infrarot- und/oder VIS- und/oder Raman-Spektren der Proben der Haare aufgenommen.

In Schritt c) wird ein Kalibriermodell erstellt, das eine Korrelation zwischen den Nahinfrarot- und/oder Infrarot- und/oder VIS- und/oder Raman-Spektren der Proben (Kalibrierspektren) und dem Gehalt an Inhaltsstoff der Proben, der mittels eines unabhängigen chromatographischen, colorimetrischen oder elektromagnetischen Verfahrens ermittelt wurde, herstellt. Das Erstellen des Kalibriermodells kann auch umfassen, dass für die Mehrzahl von Kalibrierhaarproben ein Kalibrierspektrum von zumindest einem Teil des Nahinfrarot- und/oder Infrarot- und/oder VIS- und/oder monochromatischen Lichts, welches während eines Belichtens der Kalibrierhaarprobe mit Nahinfrarot- und/oder Infrarot- und/oder VIS- und/oder monochromatischem Licht von der Kalibrierhaarprobe reflektiert und/oder gestreut wird, aufgenommen wird, ein Haarzustand der Kalibrierhaarprobe mittels eines unabhängigen chromatographischen, colorimetrischen oder elektromagnetischen Verfahrens ermittelt wird, ein Haarzustand zum Kalibrierspektrum zugewiesen wird und eine Korrelation zwischen der Mehrzahl von Kalibrierspektren und der Mehrzahl von Zuständen ermittelt wird.

Die Schritte a) und b) müssen nicht zwingend nacheinander und in dieser Reihenfolge durchgeführt werden. So kann zuerst Schritt b) und dann Schritt a) durchgeführt werden.

In einer bevorzugten Ausführungsform liegt das Kalibriermodell aus den Schritten a) bis c) als gespeicherte Information auf einem lokalen Datenträger oder in einer Cloud vor. Ein lokaler Datenträger im Sinne dieser Anmeldung umfasst alle physischen Trägersubstanzen, auf denen Daten festgehalten werden können. In einer besonders bevorzugten Ausführungsform ist der Datenträger identisch mit der Datenverarbeitungsvorrichtung an die das (N)IR, VIS-, (N)IR/VIS- oder Raman-Spektrometer zur Aufnahme von Nahinfrarot- und/oder Infrarot- und/oder VIS- und/oder Raman-Spektren der Haare angeschlossen ist. Dies kann insbesondere ein Smartphone, ein Tablet, ein Laptop, ein Smart Mirror oder ein Computer sein. In einer alternativen Ausführungsform liegt das Kalibriermodell aus den Schritten a) bis c) als gespeicherte Information in einer Cloud vor.

Durch eine Anwendung von mathematischen Modellen kann mittels Vermessung von KalibrierHaarproben, welche einen anhand eines bekannten analytischen Verfahrens ermittelten Gehalt an Haarinhaltsstoff aufweisen, ein mathematisches Modell erstellt werden, welches dann bei einer Haarprobe, auch als Tresse bezeichnet, des Individuums anhand des aufgenommenen NIR-, IR- und/oder VIS- und/oder Raman-Spektrums eine Berechnung eines Gehalts an Haarinhaltsstoff, und damit Rückschlüsse auf den Zustand der Haare erlaubt. Eine Analyse des Spektrums und eine Anwendung des Modells kann dabei beispielsweise (mit geeigneten Apps) mittels bekannter Datenverarbeitungsvorrichtungen wie Smartphones, Tablet o.ä. ausgeführt werden.

Das mathematische Modell kann ein künstliches System sein, das beispielsweise aus den KalibrierHaarproben lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden insbesondere in Verbindung mit *deep learning*-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen.

Zur Optimierung des Kalibriermodells können weitere Faktoren, insbesondere kategoriale Faktoren, wie beispielsweise Ethnizität des Individuums, Alter des Individuums (als Kategorie oder metrisch), Haarfarbe des Individuums (als Kategorie oder metrisch), berücksichtigt werden.

Im weiteren Verfahren wird in Schritt d) ein Nahinfrarot- und/oder Infrarot- und/oder VIS- und/oder Raman-Spektrum der Haare eines Individuums aufgenommen. Dies kann beispielsweise derart erfolgen, dass während eines Belichtens einer Haarprobe des Haars des Individuums mit Nahinfrarot- und/oder Infrarot- und/oder VIS-Licht und/oder monochromatischem Licht ein Spektrum von zumindest einem Teil des Nahinfrarot- und/oder Infrarot- und/oder VIS-Lichts und/oder monochromatischen Lichts, welches von der Haarprobe reflektiert und/oder gestreut wird aufgenommen wird. Vorzugsweise werden in Schritt d) mehrere Nahinfrarot- und/oder Infrarot- und/oder VIS- und/oder Raman-Spektren pro Messpunkt aufgenommen und diese jeweils gemittelt.

In Schritt e) wird der Gehalt an dem Haarinhaltsstoff der Haare des Individuums anhand des in den Schritten a) bis c) erstellten Kalibriermodells bestimmt. Aus dem ermittelten Gehalt an Haarinhaltsstoff kann der Haarzustand der abgeleitet werden. Der abgeleitete Haarzustand kann beispielsweise umfassen spröde, glanzlos, oxidativ geschädigt, hitzegeschädigt, chemisch geschädigt, trocken, feucht, fettig, hydrophil, Melanin-reich, Melanin-arm oder normal. Bei der chemischen Schädigung kann zusätzlich nach der Art der Schädigung unterschieden werden, wie beispielsweise Dauerwell-geschädigt, Straightener-geschädigt und/oder Relaxer-geschädigt.

Dazu wird beispielsweise zumindest ein Teil des Nahinfrarot- und/oder Infrarot- VIS- und/oder Raman-Spektrums vom Haar des Individuums mit dem Kalibriermodell verglichen und ein Gehalt an Haarinhaltsstoff des Haars und/oder Haarzustand ermittelt/zugewiesen.

In einer weiteren vorteilhaften Ausführungsform erfolgt die Aufnahme der Nahinfrarot- und/oder Infrarot- und/oder VIS- und/oder Raman-Spektren der Haare eines Individuums an unterschiedlichen Positionen entlang der Haare. So kann die Aufnahme der Nahinfrarot- und/oder Infrarot- und/oder VIS- und/oder Raman-Spektren am Ansatz und/oder in der Mitte und/oder an den Spitzen der Haare erfolgen. Entsprechend kann für jede dieser Positionen ein eigener Haarzustand ermittelt werden.

Die Nahinfrarotspektroskopie basiert wie andere Schwingungsspektroskopien auf der Anregung von Molekülschwingungen durch elektromagnetische Strahlung im (nahen) Infrarotbereich. Bei der Nahinfrarotspektroskopie findet die Detektion im nahen Infrarot (780-2500 nm oder ca. 12.800-4.000 cm⁻¹) statt. Im Folgenden wird für Licht mit einer Wellenzahl in einem Bereich von 12.800 bis 4000 cm⁻¹ der Begriff Nahinfrarot (NIR) und für Licht mit einer Wellenzahl in einem Bereich von 3999 bis 400 cm⁻¹ der Begriff Infrarot (IR) verwendet.

Die Nahinfrarot- (NIR-) und/oder ein Infrarot- (IR-)Spektren können beispielsweise mittels ATR-(Nah-)Infrarotspektroskopie (von Englisch "attenuated total reflection", auf Deutsch "abgeschwächte Totalreflexion") gewonnen werden.

Die VIS-Spektroskopie verwendet Licht mit einer Wellenlänge im Bereich von 380 bis 780 nm, welches auch als "sichtbares Licht" (VIS) bezeichnet wird.

Die Raman-Spektroskopie untersucht die inelastische Streuung von Licht an Molekülen oder Festkörpern (Raman-Streuung). Der Raman-Streuung entsteht durch Wechselwirkung von elektromagnetischer Strahlung und der Elektronenhülle der Moleküle und ist im Gegensatz zur (N)IR- und VIS- Spektroskopie praktisch unabhängig von der Wellenlänge der Erregerstrahlung. Bei der Raman-Spektroskopie wird die zu untersuchende Materie mit monochromatischem Licht bestrahlt, üblicherweise aus einem Laser.

In einer bevorzugten Ausführungsform des Verfahrens werden zwei oder mehr Spektroskopie-Arten verwendet. Die Aufnahme der Spektren mittels unterschiedlicher Spektroskopie-Arten kann gleichzeitig, das heißt in einem Messschritt, beispielsweise durch Spektrometer, die zwei oder mehr Spektroskopie-Arten durchführen können, erfolgen oder in zwei oder mehr Messschritten. So können beispielsweise mit einem (N)IR/VIS-Spektrometer gleichzeitig die NIR-, IR- und VIS-Spektren von Haaren aufgenommen werden. Alternativ kann beispielsweise zunächst ein NIR-Spektrometer und dann ein Raman-Spektrometer zum Einsatz kommen.

Durch Verwendung miniaturisierter (N)IR-Spektrometer, (N)IR-Sensoren, VIS-Spektrometer, VIS-Sensoren, Raman-Spektrometer, Raman-Sensoren, (N)IR/VIS-Spektrometer und/oder (N)IR/VIS-Sensoren sowie deren Anschluss an eine mobile Datenverarbeitungsvorrichtung kann das Verfahren und insbesondere Schritt d) des Verfahrens beispielsweise im privaten Bereich durch das Individuum selber, durch eine beliebige Person an einem Verkaufspunkt ("point of sale", POS) von Haarbehandlungsmitteln oder durch einen Friseur durchgeführt werden. In einer bevorzugten Ausführungsform ist die mobile Datenverarbeitungsvorrichtung ein smartes Endgerät, zum Beispiel ein Smartphone, ein Tablet oder ein Laptop.

Die (N)IR-Spektrometer, VIS-Spektrometer und/oder (N)IR/VIS-Spektrometer und/oder Raman-Spektrometer können insbesondere auch in mobiler Form bereitgestellt werden, beispielsweise in Form von Hand-Spektrometern ("handheld") oder Aufsatz-Spektrometern.

Ein Beispiel für ein geeignetes Hand-Spektrometer ist das "MicroNIR OnSite" der Firma Viavi Solutions Inc. Dieses Spektrometer wird von einem Tablet oder einem Laptop über einen USB-Anschluss mit Strom versorgt und gesteuert. Dieses Hand-Spektrometer ermöglicht mit einer Messzeit von zwischen 0,25 und 0,5 Sekunden die Aufnahme der Nahinfrarot- und/oder Infrarotspektren der Haare eines Individuums in Echtzeit. Das Spektrometer weist zwei integrierte Vakuum-Wolfram-Lampen und ein InGaAs-Photodioden-Array mit 128 Pixeln auf. Das "MicroNIR OnSite" arbeitet in einem Wellenzahlenbereich von 6060 bis 10526 cm⁻¹. Der Abstand zwischen den Haaren und dem Glas des Handspektrometers kann zwischen 0 und 15 mm betragen, wobei ein Abstand von 3 mm bevorzugt ist.

In einer Ausführungsform der Erfindung wird das ganze Verfahren zur Ermittlung einer individualisierten Haarbehandlung durch das Tablet oder den Laptop durchgeführt, welcher das "MicroNIR OnSite"-Spektrometer mit Strom versorgt und steuert. Alternativ können die gewonnenen spektroskopischen Daten an eine weitere (mobile) Datenverarbeitungsvorrichtung, insbesondere ein weiteres smartes Endgerät, gesendet werden, welches dann das Verfahren zur Ermittlung einer individualisierten Haarbehandlung durchführt. Die Übertragung der spektroskopischen Daten kann beispielsweise kabellos mittels WLAN (WiFi) oder Bluetooth erfolgen.

Ein weiteres geeignetes Hand-Spektrometer ist das "i-Spec Nano" der Firma B&W Tek. Das Spektrometer wird über einen USB-Anschluss und einer daran angeschlossenen (mobilen) Datenverarbeitungsvorrichtung oder über eine Batterie mit Strom versorgt. Das Spektrometer weist eine Lichtquelle auf und arbeitet in einem Wellenzahlenbereich von 4545 bis 7692 cm⁻¹. Die Übertragung der spektroskopischen Daten an eine (mobile) Datenverarbeitungsvorrichtung, welche dann das Verfahren zur Ermittlung einer individualisierten Haarbehandlung durchführt, kann kabellos mittels WLAN (WiFi) oder Bluetooth erfolgen.

Ebenfalls geeignet ist das NIR/VIS-Hand-Spektrometer "QualitySpec Trek" der Firma ASD Inc. Dieses arbeitet in einem Wellenzahlenbereich von 28571 bis 400 cm⁻¹ (350 - 2500 nm).

Ein weiteres geeignetes Hand-Spektrometer ist das "SCiO by Consumer Physics", welches mit Hilfe der integrierten App "SpectroScan" die spektroskopischen Daten auf einem smarten Endgerät anzeigt. Das Hand-Spektrometer arbeitet im kurzwelligen Bereich des NIR und zwar bei Wellenzahlen von 9090 bis 14285 cm⁻¹ (entspricht 700 bis 1100 nm). Die Auswertung der gemessenen Daten erfolgt mit Hilfe einer Cloud, in der beispielsweise eine Materialdatenbank, chemometrische Modelle und Algorithmen gespeichert sind.

Noch weitere geeignete Hand-Spektrometer sind von der Firma Attonics Systems erhältlich, welche entweder in Wellenzahlenbereichen von 9090 bis 26.315 cm⁻¹ (VIS-NIR) oder von 3333 bis 10.000 cm⁻¹ (NIR) arbeiten. Diese Spektrometer basieren auf Interferometern und weisen einen hohen Lichtdurchsatz und eine hohe spektrale Auflösung (< 5 nm für VIS-NIR-Spektrometer und < 20 nm für das NIR-Spektrometer) auf. Die Spektrometer weisen einen Multi-Phase Shift Array (MPA) Chip und eine optische Anordnung in einem kreisförmigen Rohr auf. Ferner sind die Spektrometer kompatibel mit mobilen Datenverarbeitungsvorrichtungen.

Weitere Beispiele für VIS-NIR-Spektrometer sind die Miniaturspektrometer "USB2000-VIS-NIR" und "USB4000-VIS-NIR" der Firma Ocean Optics. Dieses Spektrometer arbeitet mit einem Wellenlängenbereich von 350 bis 1000 nm. Die Spektrometer werden über einen USB-Anschluss mit einer Datenverarbeitungsvorrichtung verbunden werden.

Daneben existieren eine Reihe von NIR-Sensoren oder NIR-Auswertungsmodulen, welche in Hand-Spektrometer verwendet werden können. Geeignete NIR-Auswertungsmodule sind die Module "DLP® NIRscan" und "DLP® NIRscan Nano" der Firma Texas Instruments. Diese weisen zwei Wolfram-Lampen und InGaAs-Photodioden als Detektoren auf. Das Modul "DLP® NIRscan" arbeitet im Wellenzahlenbereich von 4016 bis 7407 cm⁻¹ und das Modul "DLP® NIRscan Nano" im Bereich von 5882 bis 11111 cm⁻¹. Die Kommunikation der spektroskopischen Daten erfolgt kabellos über Bluetooth Low Energy. Mit Hilfe von "Software Developer Kits" (SDK), beispielsweise das Open Source SDK von KST Technologies, können Apps entwickelt werden, die die spektroskopischen Daten auswerten oder weiter verarbeiten.

Weitere geeignete NIR-Sensoren sind unter der Bezeichnung "NeoSpectra" von Si-Ware Systems erhältlich. Konkrete Sensoren umfassen: NeoSpectra SW62221-1.7, NeoSpectra SW62221-2.1 und NeoSpectra SW62221-2.5, welche in unterschiedlichen Wellenzahlenbereichen arbeiten.

Ein geeignetes, handgehaltenes Raman-Spektrometer ist das Raman-Spektrometer "Bravo" der Firma Bruker. Das Raman-Spektrometer arbeitet in einem Spektralbereich von 300 bis 3200 cm⁻¹ und weist eine Technik zur Unterdrückung von Fluoreszenzsignalen auf. Die Anregung erfolgt mittels zweier, temperaturgesteuerten Diodenlaser. Die Übertragung der spektroskopischen Daten an eine (mobile) Datenverarbeitungsvorrichtung, welche dann das Verfahren zur Ermittlung einer individualisierten Haarbehandlung durchführt, kann kabellos mittels WLAN (WiFi) oder über ein Netzwerkkabel erfolgen.

Es ist insbesondere bevorzugt, dass ein handgehaltenes (N)IR/VIS-Spektrometer, welches die spektroskopischen Daten kabellos mittels WLAN (WiFi) oder Bluetooth überträgt, in dem Verfahren zur Ermittlung einer individualisierten Haarbehandlung eingesetzt wird.

Es kann bevorzugt sein, dass der zumindest eine Teil des Nahinfrarotlichts und/oder Infrarotlichts und/oder VIS-Lichts und/oder monochromatischen Lichts einen (Nahinfrarot-)Wellenzahlbereich von 12.500 cm⁻¹ bis 4000 cm⁻¹ (entspricht 800 bis 2.500 nm) aufweist. In diesem Bereich finden sich insbesondere die Absorptionen von flüssigem Wasser (836 nm, 970 nm, 1200 nm, 1470 nm und 1900 nm). Weitere (Schwingungs)Absorptionen von flüssigem Wasser finden sich bei 401 nm, 449 nm, 514 nm, 606 nm, 660 nm, 739 nm, 2,87 µm, 3,05 µm, 4,65 µm, 6,08 µm, 15 µm und 25 µm.

In einer bevorzugten Ausführungsform wird Schritt d) durch ein smartes Endgerät gesteuert. Dabei ist es bevorzugt, dass das Verfahren eine auf dem smarten Endgerät vorab installierte App gesteuert wird. Smarte Endgeräte umfassen insbesondere Smartphones oder Tablets.

Unter einer "App" wird im Rahmen dieser Anmeldung ein Computerprogramm bezeichnet, das genutzt wird, um eine nicht systemtechnische Funktionalität zu bearbeiten oder zu unterstützen. Der Begriff "App" umfasst insbesondere Anwendungssoftware für smarte Endgeräte wie Smartphones und Tablets ("mobile App") als auch Desktop-Anwendungssoftware. Die App kann eine native App sein, die nur auf einer Plattform funktioniert, oder eine plattformunabhängige Web-, Hybrid- oder Cross-Plattform-App.

Die App kann besonders bevorzugt über einen QR-Code, einen NFC-Chip, einen Barcode oder einen RFID-Chip direkt bei einem Friseur oder an einem Verkaufspunkt ("point of sale", POS) von Haarbehandlungsmitteln heruntergeladen werden.

Alternativ kann die App, insbesondere im Fall, dass sie auf einem smarten Endgerät installiert werden soll, über eine in das jeweilige Betriebssystem des smarten Endgeräts integrierte Internet-Vertriebsplattform heruntergeladen werden. Im Fall eines smarten Endgeräts mit dem Betriebssystem "Apple iOS" kann dies beispielsweise der "App Store" sein oder im Fall eines smarten Endgeräts mit dem Betriebssystem "Android" kann dies der "Google Play Store" sein.

In einer Ausführungsform enthält der QR-Code, der NFC-Chip, der Barcode oder der RFID-Chip einen Weblink, welcher den Anwender des Verfahrens auf eine Internetseite führt, von der der Anwender des Verfahrens die App herunterladen kann.

Es ist ferner bevorzugt, wenn der in dem Verfahren ermittelte Gehalt an Haarinhaltsstoff in einer relativen Angabe (stark erniedrigt, erniedrigt, normal, erhöht und stark erhöht) ausgegeben wird.

Alternativ oder zusätzlich kann der zugeordnete Haarzustand in einer relativen Angabe (sehr stark spröde, stark spröde, leicht spröde und normal) ausgegeben werden.

Die Ausgabe des Gehalts kann optisch, beispielsweise mittels einer Anzeigevorrichtung der (mobilen) Datenverarbeitungsvorrichtung, oder akustisch, beispielsweise mittels einer Sprachansage über einen Lautsprecher, erfolgen.

In einer besonders bevorzugten Ausführungsform des Verfahrens umfasst die individuelle Behandlungsanweisung die Empfehlung von Blondiermitteln und/oder Haarfärbemitteln und/oder Haarpflegeprodukten und/oder Stylingmitteln.

Die Empfehlung kann dabei die Anzeige oder Ansage eines konkreten Produktnamens eines Haarbehandlungsmittels, insbesondere eines Blondiermittels und/oder Haarfärbemittels und/oder Haarpflegeprodukts und/oder Stylingmittels, umfassen. Alternativ kann die Empfehlung die Anzeige oder Ansage einer Produktlinie oder -serie, insbesondere eine Blondiermittellinie/-serie und/oder Haarfärbemittellinie/-serie und/oder Haarpflegeproduktlinie/-serie und/oder Stylingmittellinie/-serie, eines Herstellers umfassen.

Werden für den Gehalt einer oder mehrerer Aminosäuren von den üblichen Werten abweichende Werte ermittelt, können beispielsweise geeignete Haarpflegeprodukte, wie Spülungen oder Kuren, empfohlen werden, welche Proteinhydrolysate enthalten. Vorzugsweise enthalten die Proteinhydrolysate die Aminosäure(n), deren Gehalt von den üblichen Werten abgewichen ist/sind.

Werden für den Gehalt eines oder mehrerer Lipide von den üblichen Werten abweichende Werte ermittelt, können beispielsweise geeignete Haarpflegeprodukte, wie Spülungen oder Kuren, empfohlen werden, welche Lipide enthalten. Vorzugsweise umfassen die in den Haarpflegeprodukten eingesetzten Lipide das/die Lipid(e), dessen/deren Gehalt von den üblichen Werten abgewichen ist.

Wird für den Gehalt von 18-Methyleicosansäure ein von den üblichen Werten abweichender Wert ermittelt, können beispielsweise geeignete Haarpflegeprodukte, wie Spülungen oder Kuren, empfohlen werden, welche kationisch modifizierte Proteine, Proteolipide und/oder kationische Silikone, enthalten. 18-Methyleicosansäure ist im Haar als Thioester gebunden und verantwortlich für den hydrophoben Schutzfilm der Haare. Ist der ermittelte Wert für 18-Methyleicosansäure niedriger als üblich, ist das Haar zu hydrophil. Durch Einsatz von kationisch modifizierten Proteinen, Proteolipiden und/oder kationischen Silikonen kann der Haarzustand hinsichtlich der Hydrophilie der Oberfläche der Haare verbessert werden.

In einer alternativen Ausführungsform des Verfahrens umfasst die individuelle Behandlungsanweisung die Empfehlung, für einen bestimmten Zeitraum von Blondierungen und/oder oxidativem Färben und/oder permanenten Verformungen und/oder Hitzebehandlungen Abstand zu nehmen.

Unter den Begriff "permanente Verformung" fallen alle Verfahren zur Lockung von glattem Haar oder Glättung von gelocktem Haar. Dieses können Dauerwell-Verfahren oder chemische Glättungsverfahren sein. Neben dem Einsatz von Chemikalien, können auch Hitzebehandlungen den Gehalt an bestimmten Haarinhaltsstoffen unerwünscht erhöhen oder erniedrigen. Entsprechend kann die individuelle Behandlungsanweisung die Empfehlung umfassen, von Hitzebehandlungen wie beispielsweise dem Einsatz von Lockenstäben oder Glätteisen, für einen bestimmten Zeitraum abzusehen.

Alternativ kann bei Durchführung des Verfahrens zur Ermittlung einer individualisierten Haarbehandlung während einer Hitzebehandlung, die individuelle Behandlungsanweisung die Empfehlung die Hitzebehandlung innerhalb eines bestimmten Zeitraums, beispielsweise sofort, zu beenden oder für einen bestimmten Zeitraum, beispielsweise einige Minuten, zu unterbrechen, umfassen. In diesem Fall kann sich die individualisierte Haarbehandlungsanweisung am ermittelten Wassergehalt der Haare orientieren.

In einer weiteren Ausführungsform der Erfindung kann die individuelle Behandlungsanweisung eine Dosierungsempfehlung für eine Blondierung und/oder eine oxidative Färbung und/oder die Vorhersage eines Resultats einer Blondierung und/oder oxidativen Färbung umfassen.

Wird der Gehalt an Melanin bestimmt, kann die individuelle Behandlungsanweisung die Empfehlung eines bezüglich des Oxidationsmittel-Gehaltes optimierten Blondiermittels oder oxidativen Färbemittels umfassen.

Bei einer Blondierung oder beim oxidativen Färben von Haaren wird in einem alkalischen Milieu die Schuppenschicht der Haare geöffnet und ein - je nach gewünschtem Blondierungs- oder Aufhellungsergebnis - unterschiedlich hoch dosierter Wasserstoffperoxid-Cocktail löst die Melanine mehr oder weniger stark auf. Das Haar wird umso heller, je höher dosiert das Wasserstoffperoxid ist. Aufgrund dieser Wirkungsweise schädigen Blondierungen oder oxidative Färbungen Haare immer spürbar. Je höher die Konzentration des Wasserstoffperoxids und je länger die Einwirkzeit ist, desto stärker ist die Schädigung. Ein in Bezug auf das gewünschtem Blondierungs- oder Aufhellungsergebnis und den Gehalt an Melanin in den Haaren des Individuums angepasstes Blondier- oder Aufhellungsmittel kann das Ausmaß der Haarschädigung reduzieren.

Umgekehrt kann bei bekannten Melaningehalt der Haare des Individuums das Resultat einer Blondierung und/oder oxidativen Färbung vorhergesagt werden. Immer mehr Anwender wünschen bevor sie eine Blondierung oder (oxidative) Färbung vornehmen einen realistischen Eindruck, wie ihre Haare nach einer Blondierung oder (oxidativen) Färbung aussehen. Viele Anbieter von Blondierungen oder (oxidativen) Färbungen bieten deshalb Farbberatungs-Apps an. Mit der App "Schwarzkopf Frisuren Styleguide" lassen sich beispielsweise Haarfarben in Echtzeit vorab testen. Dazu lädt der Anwender die App auf ein smartes Endgerät und nimmt mit der Frontkamera ein Foto seines Kopfes auf. Die Software der App erkennt Gesicht und Kopfform. Der Anwender wählt dann ein konkretes Blondierungsprodukt oder (oxidatives) Färbprodukt aus und bekommt auf der Anzeigevorrichtung des smarten Endgeräts angezeigt, wie er/sie nach Anwendung dieser Blondierung oder (oxidativen) Färbung aussieht.

Zur Vorhersage des Blondierungs- oder Färberesultat wird zunächst die Ausgangshaarfarbe bestimmt und dann basierend auf dem vom Anwender gewählten konkreten Blondierungs- oder Färbeprodukt das Blondierungs- oder Färbungsresultat berechnet. Da die Zusammensetzung von Haaren einen Einfluss auf das Blondierungs- oder Färberesultat nimmt, liefert das Verfahren zur Ermittlung einer individualisierten Haarbehandlung bessere, insbesondere realistischere, Vorhersagen eines Resultats einer Blondierung und/oder (oxidativen) Färbung.

In einer weiteren, vorteilhaften Ausführungsform des Verfahren besteht die individuelle Behandlungsanweisung darin, dem Individuum zum/vom Einsatz von Haarbehandlungsprodukten, die der Anwender des Verfahrens und/oder das Individuum anhand von QR-Codes, NFC-Chips, Barcodes oder RFID-Chips identifiziert, zu- oder abzuraten.

In dieser Ausführungsform des Verfahrens kann der Anwender des Verfahrens, beispielsweise ein Friseur oder eine beliebige Person am Verkaufspunkt von Haarbehandlungsmitteln, nach Ermittlung des Haarzustands über Bestimmung des Gehalts mindestens eines Haarinhaltsstoffes geeignete oder nicht geeignete Haarbehandlungsmittel über QR-Codes, NFC-Chips, Barcodes oder RFID-Chips, welche beispielsweise am Haarbehandlungsmittel selber oder am Ort dessen Aufbewahrung, beispielsweise am Regal im Friseurgeschäft oder im Verkaufspunkt von Haarbehandlungsmitteln, angebracht sind, ermitteln.

QR-Codes, NFC-Chips, Barcodes oder RFID-Chips ermöglichen, Informationen kabellos zu übertragen.

Als Barcode wird eine optoelektronisch lesbare Schrift bezeichnet, die aus verschieden breiten, parallelen Strichen und Lücken besteht. Die Daten in einem Strichcode werden mit optischen Lesegeräten, wie zum Beispiel Barcodelesegeräten (Scanner) oder Kameras, maschinell eingelesen und elektronisch weiterverarbeitet. Viele smarte Endgeräte weisen eine Software auf, die es ermöglicht, mit der Digitalkamera des smarten Endgeräts zu erfassen und dem Anwender die Code-Information sofort in dekodierter Form anzuzeigen.

Ein QR-Code ("Quick Response") ist ein zweidimensionaler Code, der aus einer quadratischen Matrix aus schwarzen und weißen Quadraten besteht, die die kodierten Daten binär darstellen. Üblicherweise verfügen smarte Endgeräte über eine eingebaute Kamera. Nach dem Fotografieren des QR-Codes wird mit Hilfe einer Software der QR-Code ausgelesen/interpretiert.

NFC-Chips und RFID-Chips sind Sender-Empfänger-Systeme. In diesem Fall muss mindestens ein Kommunikationspartner aktiv sein, also die Kommunikation anregen. Der andere Partner kann ein beispielsweise ein Chip ohne Energieversorgung sein. Dieser passive Teil wird auch Transponder (=Transmitter&Responder) genannt. Neben der aktiv-passiv Kommunikation zwischen beispielsweise einem smarten Endgerät als aktivem Kommunikationspartner und einem Transponder/Chip ist ebenfalls eine aktiv-aktiv Kommunikation möglich.

Die Kopplung/Anregung geschieht durch vom aktiven Kommunikationspartner erzeugte magnetische Wechselfelder mit geringer Reichweite oder durch hochfrequente Radiowellen. Damit werden nicht nur Daten übertragen, sondern auch der Transponder mit Energie versorgt. Der aktive Kommunikationspartner, beispielsweise ein smartes Endgerät, enthält eine Software, die den eigentlichen Leseprozess steuert, und eine sogenannte Middleware mit Schnittstellen zu weiteren (mobilen) Datenverarbeitungsvorrichtungen und/oder Datenbanken.

RFID ("radio-frequency identification") funktioniert über Radiowellen. Die RFID-Technik umfasst ein sehr breites Angebot an verschiedenen Chips und Lesegeräten, welche sich im Wesentlichen durch Speicherkapazität, Herstellverfahren, Kosten, Frequenzbereich und durch die Reichweite unterscheiden.

NFC ("Near Field Communication") ist eine genormte Spezialisierung der RFID-Technik, die speziell für eine Datenübertragung über kurze Distanzen (max. 10 cm) entwickelt wurde.

QR-Codes, NFC-Chips, Barcodes oder RFID-Chips können beispielsweise Informationen enthalten, für welchen Haarzustand das zugehörige Haarbehandlungsmittel geeignet oder nicht geeignet ist.

Die individuelle Behandlungsanweisung kann auch darin bestehen, die chemische Zusammensetzung eines Haarbehandlungsmittels, insbesondere eines Blondiermittels, eines Haarfärbemittels, eines Haarpflegeprodukts und/oder Haarstylingmittels zu ermitteln.

In einer alternativen Ausführungsform des Verfahrens besteht die individuelle Behandlungsanweisung darin, dem Individuum zum Einsatz von Blondiermitteln und/oder Färbemitteln und/oder Pflegeprodukten, die für das Individuum individuell hergestellt werden, zu zuraten und einen Bestellvorgang, vorzugsweise durch Aufrufen einer Internetseite eines Herstellers von individuellen Blondiermitteln und/oder individuellen Färbemitteln und/oder individuellen Pflegeprodukten, einzuleiten.

Immer mehr Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Blondiermitteln, Färbemitteln, Pflegeprodukten und Stylingmittel für Haare umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Haarzustand ungeeigneter Inhaltsstoffe vermieden werden. Umgekehrt kann die Auswahl für den ermittelten Haarzustand geeigneter Inhaltsstoffe durch den Produktkonfigurator vorgegeben oder vorgeschlagen werden.

Es ist auch bevorzugt, dass die individuelle Behandlungsanweisung gespeichert wird und in nachfolgenden Verfahren für eine langfristige Empfehlung genutzt wird.

Es kann auch bevorzugt sein, dass die individuelle Behandlungsanweisung in der Empfehlung von Nahrungsergänzungsmitteln liegt. Der Mangel oder ein geringer Gehalt an einem Haarinhaltsstoff, insbesondere an einer oder mehr Aminosäuren, kann Ursache für einen unerwünschten Haarzustand sein. So führt ein Mangel oder geringer Gehalt an Cystein, insbesondere an L-Cystein, zu sprödem Haar. L-Cystein wird in der Leber aus L-Serin und L-Methionin gebildet. Daher kann nach der Ermittlung eines geringen Gehalts an Cystein in den Haaren die individuelle Behandlungsanweisung in der Empfehlung der Aufnahme von Cystein, Serin und/oder Methionin, insbesondere der L-Formen dieser Aminosäuren, liegen.

In einer weiteren Ausführungsform findet vor der Ausgabe der Empfehlung an den Anwender ein Datenabgleich zwischen der (mobilen) Datenverarbeitungsvorrichtung, insbesondere dem smarten Endgerät, und Daten, die in einer Cloud hinterlegt sind, statt. Diese Daten können beispielsweise Daten von Anwendern mit gleichen oder ähnlichen Haarzusammensetzungen (insbesondere Gehalten an Haarinhaltsstoff) sowie gegebenenfalls weiteren gleichen oder ähnlichen Parametern (Alter, Geschlecht, Verhaltensweisen, etc.) und den daraus abgeleiteten Empfehlungen/Maßnahmen sein. Durch Aufnahme von Erfahrungswerten, beispielsweise hinsichtlich eines Behandlungserfolgs, weiterer Anwender kann die Beurteilung einer Eignung einer Behandlungsanweisung/Empfehlung bestätigt oder abgeändert werden. Damit kann ermöglicht sein, dass der Anwender immer eine optimale Empfehlung erhält.

In verschiedenen Ausführungsbeispielen kann die individuelle Behandlungsanweisung die Empfehlung umfassen, einen Friseur aufzusuchen. In verschiedenen Ausführungsbeispielen kann direkt über die Software/App, welche die individuelle Behandlungsanweisung ermittelt, ein Buchungsvorgang eingeleitet werden. Dazu können beispielsweise in der Software/App die Kontaktdaten von Friseuren hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Friseurtermins über eine separate Software/App, wie beispielsweise Treatwell, erfolgen.

In einer besonders bevorzugten Ausführungsform wird zusätzlich zur Bestimmung eines Haarinhaltsstoffes der Haare deren Kräuselung bestimmt wird und diese bei der individuellen Behandlungsanweisung berücksichtigt.

In einer ebenfalls bevorzugten Ausführungsform wird zusätzlich zur Bestimmung eines Haarinhaltsstoffes der Haare deren Dicke bestimmt wird und diese bei der individuellen Behandlungsanweisung berücksichtigt.

Neben dem Gehalt an Inhaltsstoff der Haare haben weitere Eigenschaften der Haare Einfluss auf den Erfolg einer Behandlung der Haare. So können insbesondere die Kräuselung und/oder die Dicke von Haaren einen Einfluss auf den Erfolg einer Behandlung der Haare nehmen. Die Kräuselung und/oder die Dicke von Haaren nimmt insbesondere Einfluss auf die Behandlung von Haaren mit Pflegemitteln.

Die Werte für die Kräuselung und/oder Dicke von Haaren können beispielsweise mit geeigneten Verfahren durch das smarte Endgerät, auf welchem das Verfahren zur Ermittlung einer individualisierten Haarbehandlung durchgeführt wird, ermittelt werden.

Die Kräuselung von Haaren kann beispielsweise mit Hilfe von Bildbearbeitungs- und Bildverarbeitungsverfahren bestimmt werden. Dazu fotografiert der Anwender des Verfahrens zumindest einen Teil der Haare des Individuums. Geeignete Bildbearbeitungs- und Bildverarbeitungsprogramme wie "ImageJ" bestimmen mit Hilfe geeigneter Plug-Ins den linearen Anteil im Bild. Glattes Haar führt zu einem hohen Anteil an Linearität, stark gekräuseltes Haar zu einem geringen Anteil an Linearität. Der Kräuselungsgrad kann bevorzugt in "% Kräuselung" angegeben werden.

Das Bildbearbeitungs- und Bildverarbeitungsprogramm zur Bestimmung der Kräuselung kann Bestandteil der App zur Durchführung des Verfahrens zur individualisierten Haarbehandlung sein. Alternativ kann die Bestimmung der Kräuselung mit Hilfe von Bildbearbeitungs- und Bildverarbeitungsverfahren mittels separater Verfahren durchgeführt werden. Vorteilhaft wird das separate Verfahren durch eine App, welche sich auch auf der mobilen Datenverarbeitungsvorrichtung, insbesondere dem smarten Endgerät, welche zur Durchführung des Verfahrens zur individualisierten Haarbehandlung eingesetzt wird, durchgeführt.

Die Bestimmung der Kräuselung kann über weitere separate Verfahren, welche nicht mit dem Verfahren zur Ermittlung einer individualisierten Haarbehandlung in Verbindung stehen, oder auch empirisch erfolgen.

Die Information über die Kräuselung kann über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei relative Kräuselungsgrade, wie beispielsweise "gar nicht", "schwach", "leicht", "mittel", "stark" und "sehr stark", vorgeben und der Anwender des Verfahrens wählt die subjektiv bestimmte Kräuselung aus. Im Fall einer durch ein separates Verfahren bestimmten, prozentualen Kräuselung, kann ein konkreter Zahlenwert, beispielsweise "20 %", eingegeben werden.

Die Dicke kann beispielsweise mit Hilfe eines Zubehörs für smarte Endgeräte bestimmt werden. Dazu wird beispielsweise ein Mikroskop-Aufsatz über die Linse des smarten Endgeräts geklemmt. Beispiele für solche Mikroskop-Aufsätze für smarte Endgeräte sind das "Nurugo Micro" der Firma Nurugo oder "µPeek" des Herstellers Scrona. Der Anwender des Verfahrens bestimmt vor oder nach der Aufnahme von Nahinfrarot- und/oder Infrarot- und/oder VIS- und/oder Raman-Spektren der Haare des Individuums (Schritt d) auch die Dicke der Haare des Individuums. Dazu fotografiert er mit Hilfe eines Mikroskop-Aufsatzes 2 bis 20, vorzugsweise 3 bis 15 und insbesondere bevorzugt 5 bis 10, verschiedene Haare zusammen mit einer Größenreferenz. Mit einer Auswertungssoftware, welche in der App zur Durchführung des Verfahrens zur individualisierten Haarbehandlung integriert sein kann, wird die durchschnittliche Haardicke des Individuums bestimmt.

Ein weiteres, alternatives Verfahren zur Bestimmung der Haardicke, welches mit geeignetem Zubehör durch ein smartes Endgerät durchgeführt werden kann, beinhaltet die Beugung von Laserlicht.

Die Bestimmung der Haardicke kann über separate Verfahren, welche nicht mit dem Verfahren zur Ermittlung einer individualisierten Haarbehandlung in Verbindung stehen, oder auch empirisch erfolgen.

Die Information über die Haardicke kann über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei relative Haardicken, wie beispielsweise "dünn", "normal" und "dick", vorgeben und der Anwender des Verfahrens wählt die subjektiv bestimmte Haardicke aus. Im Fall einer durch ein separates Verfahren bestimmten, absoluten Haardicke, kann ein Zahlenwert, beispielsweise 80 µm, eingegeben werden.

Dicke Haare und (sehr) stark gekräuselte Haare benötigen zur optimalen Pflege Haarpflegeprodukte mit einem hohen Anteil an Fett- oder Öl-haltigen Inhaltsstoffen, während für dünne und/oder glatte Haare Haarpflegeprodukte mit einem niedrigen Anteil an Fett- oder Öl-haltigen Inhaltsstoffen vorteilhaft sind.

Der Einsatz von Wasserstoffperoxid in einem stark alkalischen Milieu führt bei Blondierungen und oxidativen Färbungen in den meisten Fällen zu einer Schädigung der so behandelten Haare. Um eine Schädigung zu vermeiden oder zu reduzieren, enthalten Mittel zur Blondierung und/oder zum oxidativen Färben oftmals selber auch Pflegestoffe.

In einer besonders bevorzugten Ausführungsform des Verfahrens umfasst die individuelle Behandlungsanweisung in Abhängigkeit des ermittelten Haarzustands der Haare und in Abhängigkeit der Dicke und/oder der Kräuselung die Empfehlung von Blondiermitteln und/oder Haarfärbemitteln und/oder Haarpflegeprodukten mit einem auf den Haarzustand der Haare und die Dicke und/oder Kräuselung der Haare abgestimmten Gehalt an Fett- oder Öl-haltigen Inhaltsstoffen.

Fett- und Öl-haltige Inhaltsstoffe umfassen insbesondere Glycerinmono-, -di- oder -triester mit Fettsäuren, Fettsäuren, Fettalkohole, Fettsäuremono- oder-diester mit Fettalkoholen, Pflanzenöle, mineralische Öle, natürliche Wachse und synthetische Wachse.

Eine weitere wichtige Eigenschaft der Haare, die Einfluss auf den Erfolg einer Behandlung der Haare nimmt, ist der Grauanteil. Der Grauanteil von Haaren hat insbesondere eine Auswirkung auf das Ergebnis einer Blondierung oder eines oxidativen Färbeprozesses.

Die Bestimmung des Grauanteils kann über separate Verfahren, welche nicht mit dem Verfahren zur Ermittlung einer individualisierten Haarbehandlung in Verbindung stehen, oder auch empirisch erfolgen.

Die Bestimmung des Grauanteils kann beispielsweise mit Hilfe von Bildbearbeitungs- und Bildverarbeitungsverfahren bestimmt werden. Dazu fotografiert der Anwender des Verfahrens zumindest einen Teil der Haare des Individuums. Vorzugsweise beinhaltet der fotografierte Teil der Haare weite Teile des Ansatzes. Geeignete Bildbearbeitungs- und Bildverarbeitungsprogramme bestimmen den Grauanteil im Bild. In einer besonders bevorzugten Ausführungsform ist die Bestimmung des Grauanteils integraler Bestandteil des Verfahrens zur individualisierten Haarbehandlung und wird von der App, welche das Verfahren zur Ermittlung einer individualisierten Haarbehandlung durchführt, durchgeführt.

Die Information über den Grauanteil kann auch über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei prozentuale Grauanteile, wie beispielsweise "10%", "30%", "50%", "70%", "90%" und "100%" vorgeben und der Anwender des Verfahrens wählt den subjektiv bestimmten Grauanteil aus. Im Fall eines durch ein separates Verfahren bestimmten, prozentualen Grauanteils, kann ein Zahlenwert, beispielsweise "68 %", eingegeben werden.

Graue Haare nehmen die beim oxidativ Färben eingesetzten Oxidationsfarbstoffvorprodukte meist schlechter auf als pigmentreichere Haare. Dies führt je nach Grauanteil der Haare zu unterschiedlichen Färberesultaten.

Bei Individuen mit einem schlechten Haarzustand, insbesondere Individuen mit (sehr) stark oxidativ geschädigtem Haar, und keinem oder geringen Grauanteil kann deshalb die individuelle Behandlungsanweisung darin bestehen, dem Individuum vom Einsatz von Blondiermitteln und/oder Färbemitteln abzuraten und zum Einsatz von Färbungen ohne oxidative Behandlung, beispielsweise Tönungen oder Intensiv-Tönungen zu raten.

## Patentansprüche

1. Verfahren zur individualisierten Behandlung von Haaren, **gekennzeichnet durch** die Schritte
a) Ermitteln eines Gehalts an Haarinhaltsstoff mehrerer Proben von Haaren mit einem unterschiedlichen Haarzustand mittels eines chromatographischen oder colorimetrischen Verfahrens;
b) Aufnahme von Nahinfrarot- und/oder Infrarot- und/oder VIS- und/oder Raman-Spektren der Proben von Haaren mit einem unterschiedlichen Haarzustand;
c) Erstellen eines Kalibriermodells, das eine Korrelation zwischen Nahinfrarot- und/oder Infrarot- und/oder VIS- und/oder Raman-Spektren und dem Gehalt an Haarinhaltsstoff herstellt;
d) Aufnahme von einem Nahinfrarot- und/oder Infrarot- und/oder VIS- und/oder Raman-Spektrum der Haare eines Individuums;
e) Bestimmung eines Haarzustands der Haare dieses Individuums anhand des Kalibriermodells; und
f) Ausgabe einer individuellen Behandlungsanweisung zu den Haaren des Individuums in Abhängigkeit vom ermittelten Haarzustand,
wobei der Haarinhaltsstoff ausgewählt ist aus der Gruppe bestehend aus Aminosäuren, Lipiden, Melaninen, Wasser und Mischungen davon.

2. Verfahren nach Anspruch 1, **dadurch gekennzeichnet, dass** das Kalibriermodell aus den Schritten a) bis c) als gespeicherte Information auf einem lokalen Datenträger oder in einer Cloud vorliegt.

3. Verfahren nach einem der Ansprüche 1 oder 2, **dadurch gekennzeichnet, dass** Schritt d) bei einem Friseur, an einem Verkaufspunkt ("point of sale", POS) von Haarbehandlungsmitteln oder im privaten Bereich durchgeführt wird.

4. Verfahren nach einem der Ansprüche 1 bis 3, **dadurch gekennzeichnet, dass** Schritt d) durch ein smartes Endgerät gesteuert wird, vorzugsweise über eine vorab installierte App, welche besonders bevorzugt über einen QR-Code, einen NFC-Chip, einen Barcode oder einen RFID-Chip direkt bei einem Friseur oder an einem Verkaufspunkt ("point of sale", POS) von Haarbehandlungsmitteln heruntergeladen werden kann.

5. Verfahren nach einem der Ansprüche 1 bis 4, **dadurch gekennzeichnet, dass** der Haarzustand ein Haarzustand ausgewählt aus der Gruppe spröde, glanzlos, oxidativ geschädigt, hitzegeschädigt, chemisch geschädigt, trocken, feucht, fettig, hydrophil, Melanin-reich, Melanin-arm, normal und Kombinationen davon ist.

6. Verfahren nach einem der Ansprüche 1 bis 5, **dadurch gekennzeichnet, dass** das Kalibriermodell weitere Faktoren, insbesondere kategoriale Faktoren, beispielsweise Ethnizität des Individuums, Alter des Individuums und/oder Haarfarbe des Individuums, berücksichtigt.

7. Verfahren nach einem der Ansprüche 1 bis 6, **dadurch gekennzeichnet, dass** die individuelle Behandlungsanweisung die Empfehlung von Haarbehandlungsmitteln, insbesondere Blondiermitteln und/oder Haarfärbemitteln und/oder Haarpflegeprodukten und/oder Stylingmitteln, umfasst.

8. Verfahren nach einem der Ansprüche 1 bis 7, **dadurch gekennzeichnet, dass** die individuelle Behandlungsanweisung die Empfehlung umfasst, für einen bestimmten Zeitraum von Blondierungen und/oder oxidativem Färben und/oder permanenten Verformungen und/oder Hitzebehandlung Abstand zu nehmen.

9. Verfahren nach einem der Ansprüche 1 bis 7, **dadurch gekennzeichnet, dass** die individuelle Behandlungsanweisung die Empfehlung umfasst, für einen bestimmten Zeitraum die Haarfarbe nur um eine maximale Zahl von Nuancen aufzuhellen und/oder oxidativ zu färben.

10. Verfahren nach einem der Ansprüche 1 bis 7, **dadurch gekennzeichnet, dass** die individuelle Behandlungsanweisung eine Dosierungsempfehlung für eine Blondierung und/oder eine oxidative Färbung und/oder die Vorhersage eines Resultats einer Blondierung und/oder oxidativen Färbung umfasst.

11. Verfahren nach einem der Ansprüche 1 bis 10, **dadurch gekennzeichnet, dass** die individuelle Behandlungsanweisung darin besteht, dem Individuum zum/vom Einsatz von Haarbehandlungsprodukten, die das Individuum anhand von QR-Codes, NFC-Chips, Barcodes oder RFID-Chips, identifiziert, zu- oder abzuraten.

12. Verfahren nach einem der Ansprüche 1 bis 10, **dadurch gekennzeichnet, dass** die individuelle Behandlungsanweisung darin besteht, dem Individuum zum Einsatz von Blondiermitteln und/oder Haarfärbemitteln und/oder Haarpflegeprodukten, die für das Individuum individuell hergestellt werden, zu zuraten und einen Bestellvorgang, vorzugsweise durch Aufrufen einer Internetseite eines Herstellers von individuellen Blondiermitteln und/oder individuellen Haarfärbemitteln und/oder individuellen Haarpflegeprodukten und/oder individuellen Haarstylingmitteln, einzuleiten.

13. Verfahren nach einem der Ansprüche 1 bis 12, **dadurch gekennzeichnet, dass** zusätzlich zum Haarzustand der Haare deren Kräuselung bestimmt wird und diese bei der individuellen Behandlungsanweisung berücksichtigt wird.

14. Verfahren nach einem der Ansprüche 1 bis 13, **dadurch gekennzeichnet, dass** zusätzlich zum Haarzustand der Haare deren Dicke bestimmt wird und diese bei der individuellen Behandlungsanweisung berücksichtigt wird.

15. Verfahren nach einem der Ansprüche 1 bis 14, **dadurch gekennzeichnet, dass** zusätzlich zum Haarzustand der Haare deren Grauanteil bestimmt wird und diese bei der individuellen Behandlungsanweisung berücksichtigt wird.

## Claims

1. A method for individualized treatment of hair, **characterized by** the steps of:
a) detecting a content of hair constituents of a plurality of samples of hair having a different hair condition by means of a chromatographic or colorimetric method;
b) recording near-infrared and/or infrared and/or VIS and/or Raman spectra of the samples of hair having a different hair condition;
c) creating a calibration model which establishes a correlation between near-infrared and/or infrared and/or VIS and/or Raman spectra and the content of hair constituents;
d) recording a near-infrared and/or infrared and/or VIS and/or Raman spectrum of the hair of an individual;
e) determining a hair condition of the hair of said individual on the basis of the calibration model; and
f) outputting individual treatment instructions for the hair of the individual depending on the detected hair condition,
the hair constituents being selected from the group consisting of amino acids, lipids, melanin, water and mixtures thereof.

2. The method according to claim 1, **characterized in that** the calibration model from steps a) to c) is present as stored information on a local data carrier or in a cloud.

3. The method according to one of claims 1 or 2, **characterized in that** step d) is carried out at a hairdresser, at a point of sale (POS) of hair treatment agents or in the private sector.

4. The method according to one of claims 1 to 3, **characterized in that** step d) is controlled by a smart terminal, preferably via a previously installed app, which particularly preferably can be downloaded directly at a hairdresser or at a point of sale (POS) of hair treatment agents by means of a QR code, an NFC chip, a bar code or an RFID chip.

5. The method according to one of claims 1 to 4, **characterized in that** the hair condition is a hair condition selected from the group of brittle, dull, oxidatively damaged, heat-damaged, chemically damaged, dry, damp, greasy, hydrophilic, melanin-rich, low in melanin, normal and combinations thereof.

6. The method according to one of claims 1 to 5, **characterized in that** the calibration model takes into account further factors, in particular categorical factors, for example ethnicity of the individual, age of the individual and/or hair color of the individual.

7. The method according to one of claims 1 to 6, **characterized in that** the individual treatment instructions comprise the recommendation of hair treatment agents, in particular bleaching agents and/or hair coloring agents and/or hair care products and/or styling agents.

8. The method according to one of claims 1 to 7, **characterized in that** the individual treatment instructions comprise the recommendation to refrain from bleaching and/or oxidative dyeing and/or permanent shaping and/or heat treatment for a certain period of time.

9. The method according to one of claims 1 to 7, **characterized in that** the individual treatment instructions comprise the recommendation to lighten and/or oxidatively dye the hair color only by a maximum number of shades for a certain period of time.

10. The method according to one of claims 1 to 7, **characterized in that** the individual treatment instructions comprise a dosage recommendation for bleaching and/or oxidative coloring and/or the prediction of a result of bleaching and/or oxidative coloring.

11. The method according to one of claims 1 to 10, **characterized in that** the individual treatment instructions consist in advising the individual to use hair treatment products which the individual identifies using QR codes, NFC chips, bar codes or RFID chips, or advising the individual against using said products.

12. The method according to one of claims 1 to 10, **characterized in that** the individual treatment instructions consist in advising the individual on the use of bleaching agents and/or hair coloring agents and/or hair care products that are individually prepared for the individual, and initiating an ordering process, preferably by opening a website of a manufacturer of individual bleaching agents and/or individual hair coloring agents and/or individual hair care products and/or individual hair styling agents.

13. The method according to one of claims 1 to 12, **characterized in that**, in addition to the hair condition of the hair, the curl thereof is determined and said curl is taken into account in the individual treatment instructions.

14. The method according to one of claims 1 to 13, **characterized in that**, in addition to the hair condition of the hair, the thickness thereof is determined and said thickness is taken into account in the individual treatment instructions.

15. The method according to one of claims 1 to 14, **characterized in that**, in addition to the hair condition of the hair, the proportion of gray thereof is determined and said proportion is taken into account in the individual treatment instructions.

## Revendications

1. Procédé de traitement personnalisé des cheveux, **caractérisé par** les étapes suivantes :
a) détermination d'une teneur en constituant des cheveux de plusieurs échantillons de cheveux ayant un état des cheveux différent au moyen d'une méthode chromatographique ou colorimétrique ;
b) enregistrement des spectres infrarouges proches et/ou infrarouges et/ou visibles et/ou Raman des échantillons de cheveux ayant un état des cheveux différent ;
c) création d'un modèle d'étalonnage qui établit une corrélation entre les spectres infrarouges proches et/ou infrarouges et/ou visibles et/ou Raman et la teneur en constituant des cheveux ;
d) enregistrement des spectres infrarouges proches et/ou infrarouges et/ou visibles et/ou Raman des cheveux d'un individu ;
e) détermination de l'état des cheveux de cet individu sur la base du modèle d'étalonnage ; et
f) fourniture d'une indication individuelle de traitement pour les cheveux de l'individu en fonction de l'état des cheveux déterminé,
le constituant des cheveux étant choisi dans le groupe constitué des acides aminés, des lipides, des mélanines, de l'eau et de mélanges de ceux-ci.

2. Procédé selon la revendication 1, **caractérisé en ce que** le modèle d'étalonnage des étapes a) à c) se présente sous forme d'informations stockées sur un support de données local ou dans un nuage.

3. Procédé selon l'une des revendications 1 ou 2, **caractérisé en ce que** l'étape d) est réalisée chez un coiffeur, dans un point de vente (POS) de produits de traitement capillaire ou dans un cadre privé.

4. Procédé selon l'une des revendications 1 à 3, **caractérisé en ce que** l'étape d) est commandée par un terminal intelligent, de préférence au moyen d'une application installée au préalable, qui peut être directement téléchargée de manière particulièrement préférée chez un coiffeur ou dans un point de vente (POS) de produits de traitement capillaire à l'aide d'un code QR, d'une puce NFC, d'un code-barres ou d'une puce RFID.

5. Procédé selon l'une des revendications 1 à 4, **caractérisé en ce que** l'état des cheveux est un état des cheveux choisi dans le groupe des cheveux cassants, ternes, endommagés par oxydation, endommagés par la chaleur, chimiquement endommagés, secs, humides, gras, hydrophiles, riches en mélanine, pauvres en mélanine, normaux et des combinaisons de ceux-ci.

6. Procédé selon l'une des revendications 1 à 5, **caractérisé en ce que** le modèle d'étalonnage prend en compte d'autres facteurs, notamment des facteurs de catégorie, par exemple l'ethnie de l'individu, l'âge de l'individu et/ou la couleur des cheveux de l'individu.

7. Procédé selon l'une des revendications 1 à 6, **caractérisé en ce que** l'indication individuelle de traitement comprend la recommandation d'agents de traitement capillaire, en particulier d'agents décolorants et/ou de colorants capillaires et/ou de produits de soins capillaires et/ou d'agents coiffants.

8. Procédé selon l'une des revendications 1 à 7, **caractérisé en ce que** l'indication individuelle de traitement comprend la recommandation d'éviter toute décoloration et/ou coloration par oxydation et/ou permanente et/ou traitement thermique pendant une certaine période.

9. Procédé selon l'une des revendications 1 à 7, **caractérisé en ce que** l'indication individuelle de traitement comprend la recommandation d'éclaircir et/ou de colorer par oxydation la couleur des cheveux d'un nombre maximum de nuances uniquement pendant une certaine période.

10. Procédé selon l'une des revendications 1 à 7, **caractérisé en ce que** l'indication individuelle de traitement comprend une recommandation de dosage pour une décoloration et/ou une coloration par oxydation et/ou la prévision d'un résultat d'une décoloration et/ou d'une coloration par oxydation.

11. Procédé selon l'une des revendications 1 à 10, **caractérisé en ce que** l'indication individuelle de traitement consiste à recommander ou à déconseiller à l'individu l'utilisation de produits de traitement capillaire, que l'individu identifie à l'aide de codes QR, de puces NFC, de codes-barres ou de puces RFID.

12. Procédé selon l'une des revendications 1 à 10, **caractérisé en ce que** l'indication individuelle de traitement consiste à recommander à l'individu l'utilisation d'agents décolorants et/ou de colorants capillaires et/ou de produits de soins capillaires, qui sont fabriqués de manière personnalisée pour l'individu et à initier un processus de commande, de préférence en ouvrant le site Internet d'un fabricant d'agents décolorants personnalisés et/ou de colorants capillaires personnalisés et/ou de produits de soins capillaires personnalisés et/ou d'agents coiffants personnalisés.

13. Procédé selon l'une des revendications 1 à 12, **caractérisé en ce qu'**en plus de l'état des cheveux, leur frisure est déterminée et elle est prise en compte dans l'indication individuelle de traitement.

14. Procédé selon l'une des revendications 1 à 13, **caractérisé en ce qu'**en plus de l'état des cheveux, leur épaisseur est déterminée et elle est prise en compte dans l'indication individuelle de traitement.

15. Procédé selon l'une quelconque des revendications 1 à 14, **caractérisé en ce qu'**en plus de l'état des cheveux, la proportion de gris est déterminée et elle est prise en compte dans l'indication individuelle de traitement.
